(19) Europäisches Patentamt
European Patent Office
Office européen des brevets

(11) **EP 4 318 016 A1**

(12) **EUROPEAN PATENT APPLICATION**

(43) Date of publication:
**07.02.2024 Bulletin 2024/06**

(21) Application number: **22188017.2**

(22) Date of filing: **01.08.2022**

(51) International Patent Classification (IPC):
*G01R 33/565* (2006.01)    *G01R 33/56* (2006.01)

(52) Cooperative Patent Classification (CPC):
**G01R 33/56509; G01R 33/5608; G06N 3/0464; G06N 3/08**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Koninklijke Philips N.V.**
**5656 AG Eindhoven (NL)**

(72) Inventors:
• **SOMMER, Karsten**
  **Eindhoven (NL)**

• **KEUPP, Jochen**
  **Eindhoven (NL)**
• **SCHUELKE, Christophe Michael Jean**
  **5656 AG Eindhoven (NL)**
• **PEZZOTTI, Nicola**
  **5656 AG Eindhoven (NL)**

(74) Representative: **Philips Intellectual Property & Standards**
**High Tech Campus 52**
**5656 AG Eindhoven (NL)**

(54) **NEURAL NETWORK GUIDED MOTION CORRECTION IN MAGNETIC RESONANCE IMAGING**

(57) Described herein is a medical system (100, 300) comprising a memory (110) storing machine executable instructions (120) and a motion estimating neural network (122, 700, 800, 900, 1000) configured for outputting trajectory data (130) in response to receiving a trial motion trajectory (128) as input. The execution of the machine executable instructions causes a computational system (104) to: receive (200) measured k-space data (124) descriptive of a subject (318); perform (202) motion estimation of the subject between the sequence of discrete acquisitions by solving an optimization problem to determine a calculated motion trajectory of the subject in the predefined coordinate system, wherein the optimization problem is modified using the trajectory data; and reconstruct (204) a final motion corrected magnetic resonance image (136) from the measured k-space data and the calculated motion trajectory in the predefined coordinate system.

EP 4 318 016 A1

```
┌─────────────────────────────────────────────────┐
│ receive measured k-space data descriptive of a subject │──200
└─────────────────────────────────────────────────┘
                        │
┌─────────────────────────────────────────────────┐
│ perform motion estimation of the subject between the
│ sequence of discrete acquisitions by solving an
│ optimization problem to determine a calculated motion
│ trajectory of the subject in a predefined
│ coordinate system                                │──202
└─────────────────────────────────────────────────┘
                        │
┌─────────────────────────────────────────────────┐
│ reconstruct a final motion corrected MR image
│ from the measured k-space data and the
│ calculated motion trajectory in
│ the predefined coordinate system                 │──204
└─────────────────────────────────────────────────┘
```

Fig. 2

## Description

FIELD OF THE INVENTION

The invention relates to magnetic resonance imaging, in particular to motion corrected magnetic resonance imaging.

BACKGROUND OF THE INVENTION

A large static magnetic field is used by Magnetic Resonance Imaging (MRI) scanners to align the nuclear spins of atoms as part of the procedure for producing images within the body of a patient. This large static magnetic field is referred to as the B0 field or the main magnetic field. Various quantities or properties of the subject can be measured spatially and imaged using MRI. A difficulty with performing magnetic resonance imaging is that it takes time to acquire the k-space data and often the subject will move causing blurring and motion artifacts in the resulting magnetic resonance image.

The journal publication Lundervold et. al., 'An overview of deep learning in medical imaging focusing on MRI,' Z Med Phys 29 (2019) 102-127, is a review article that discusses how deep learning has been applied to the MRI processing chain. This includes acquisition to retrieval, image segmentation and disease prediction.

SUMMARY OF THE INVENTION

The invention provides for a medical system, a computer program, and a method in the independent claims. Embodiments are given in the dependent claims.

As was mentioned above, subject motion during the acquisition of k-space data during an MRI acquisition can lead to blurring and image artifacts. There are a variety of ways to correct for subject motion. One technique is determining the subject motion by formulating a motion estimation problem as a numerical optimization problem. Initially, the subject's motion can be assumed, and a trial image is constructed. This trial image can be resampled back into k-space and compared to the originally measured k-space data. Normal optimization techniques can be used to numerically search for the subject motion to minimize the difference between the resampled k-space data and the measured k-space data. A difficulty in this approach is that it can be very numerically intensive.

There is also the risk of settling into a local minimum and not properly solving the optimization problem. Embodiments may provide for a means of accelerating the numerical search as well as to minimize the probability of settling into a local minimum by modifying the optimization problem using a motion estimating neural network. In one example the optimization is modified by adding a term to the optimization problem's cost function which is a function of a probability that the trial motion trajectory is correct. The effect of this is that the numerical algorithm is guided to the most likely solutions to subject motion, and this may be done more rapidly. A motion estimating neural network is configured to provide the trajectory probability in response to receiving the trial motion trajectory as input.

In another example the motion estimating neural network is configured to output a suggested motion trajectory in response to receiving the trial motion trajectory as input. The suggested motion trajectory is then used to modify or adjust the trial motion trajectory either before or after an iteration of solving the optimization problem.

In one aspect the invention provides for a medical system that comprises a memory storing machine-executable instructions and a motion estimating neural network. The motion estimating neural network is configured for outputting trajectory data in response to receiving a trial motion trajectory as input. The trial motion trajectory is essentially a proposed motion trajectory of a subject or a motion trajectory of the subject in one iteration of an optimization problem. The trial motion trajectory has a predefined coordinate system. The predefined coordinate system would likely be defined in terms of the motion problem itself. For example, if one were dealing with the rigid motion of a subject's skull during the magnetic resonance imaging, it may be coordinates related to the rotation and motion of the skull. In other instances, it may be a vector field which defines the motion of the subject as a non-rigid motion. Below an optimization problem is described and likely the predefined coordinate system would be the coordinate system of the optimization problem.

The medical system further comprises a computational system. Execution of the machine-executable instructions causes the computational system to receive measured k-space data descriptive of a subject. The measured k-space data is divided into a sequence of discrete acquisitions. Normally when magnetic resonance imaging data is acquired it is acquired in shots or groups of k-space data. The measured k-space data represents these measured acquisitions or shots of magnetic resonance imaging data. A likely cause of image degradation and artifacts during magnetic resonance imaging is the movements of a subject in between the discrete acquisitions.

Execution of the machine-executable instructions further causes the computational system to perform motion estimation of the subject between the sequence of discrete acquisitions by solving an optimization problem to determine a calculated motion trajectory of the subject in the predefined coordinate system. The optimization problem is formulated to minimize the difference between the measured k-space data and a transformation of re-sampled k-space data of a motion-corrected trial magnetic resonance image as a function of a trial motion trajectory and the measured k-space data. Performing the motion estimation comprises receiving the trajectory data in response to inputting the trial motion trajectory into the motion estimating neural network. The optimization problem is modified using the trajectory data from the motion estimating neural network. The solution of motion of the subject using an optimization problem is one technique that has been used previously. The difference is that the optimization problem is modified by the probability data.

Normally, when solving an optimization problem, trajectories are iteratively refined to minimize the difference between the measured k-space data and the re-sampled k-space data (data consistency), either by naive sampling of the trajectory space or by gradient descent of the data consistency term. A difficulty with this approach is that it can be very computationally intensive and slow to converge. The motion estimating neural network can be used to guide the optimization problem so that it follows or goes to the most likely solution. For example, during a particular type of magnetic resonance imaging examination there may be motions which are very typical of a subject. For example, breathing motion or coughing may lead to particular types of motion in a subject. Instead of searching the entire solution space the motion estimating neural network is used to guide or heavily favor more likely solutions during the solving of the optimization problem. This may result in a greatly reduced computational burden.

Execution of the machine-executable instructions further causes the computational system to reconstruct a final motion-corrected magnetic resonance image from the k-space data and the calculated motion trajectory in the predefined coordinate system. This final reconstruction may be performed after the motion estimation is complete or it may be performed in the course of performing the motion estimation. For example, the solution for the calculated motion trajectory may involve the calculation of trial images. Once the optimal solution is found or the optimization problem ends, the final or the best image may be used and assigned as the final motion-corrected magnetic resonance image.

In another embodiment, the trajectory data comprises trajectory probability data, wherein the optimization problem comprises a cost function that is a function of the trajectory probability. The optimization problem then comprises a cost function that is a function of the trajectory probability. The function of the motion estimating neural network is to provide this probability which works as a weighting function.

Three specific examples are described below:

1) Segmented trajectory parametrization

Assuming a rigid motion model, a generalized motion-compensated reconstruction may be formulated as follows:

$$\min_{T} \|AFSTx - y\|$$

Where y is the acquired k-space data, x the motion-free image, A a sampling matrix, F the Discrete Fourier Transform, S the coil sensitivity matrix and T the rigid motion transformation matrix.

The patient's motion trajectory is assumed to be partitioned into segments by a set of time points $\tau = \{t_i\}_{i=1}^{n}$ that satisfy the condition

$$t_1 < t_2 < \cdots < t_n$$

A piece-wise approximation of the patient's motion trajectory is realized using a generic functional form for each segment, e.g. using an m-th order polynomial,

$$\theta(t) = \sum_{i=1}^{n-1}\left(\left.\sum_{j=0}^{m}\alpha_{ij}t^j\right|_{t_i<t<t_{i+1}}\right)$$

where $\alpha_{ij}$ is the j-th order coefficient of the i-th segment.
The motion trajectory can then be fully described by combining these coefficients as well as the time partitioning into a single vector,

$$K = (K_1, \ldots, K_n)^T$$

where $K_i = (t_i, \alpha_{i0}, \ldots, \alpha_{im})$.
The motion estimation problem can then be reformulated as

$$\min_{K}\frac{1}{2}\left\|AFST\big(\theta(K)\big)x - y\right\|_2^2 + \lambda\log p(K)$$

where the probability distribution p(K) is given by the neural network, which uses the patient and scan metadata as input variables. This way, motion estimation is simplified by performing the optimization in a low-dimensional search space spanned by K.
The regularization parameter λ defines the influence of the trajectory probability on the optimization problem: the larger λ, the more the search will be constrained to high-probability regions of the search space. In practice, λ can increased over time as more available training data improves probability predictions.
Note that the polynomial form of the motion trajectory is only used as an example here, and in practice a variety of functional forms can be used.
The impact of the individual metadata that is fed to the neural network on the motion trajectory may possibly vary greatly the probabilities. To account for this variability, the input data is first processed by one or several fully connected layers that encode the information content into a latent code. Optionally this code is then further processed using ID convolutional layers, and the required output data ($K_i$ coefficients in this example) is again generated using fully connected layer(s). To avoid overfitting, the depth of the network and size of the individual layers is chosen depending on the size of the available dataset.

Once a certain performance criterion is reached, the parameters of the trained network (i.e. its weights) can then be shared with other sites to allow for a distributed learning setup. This way, data from diverse patient groups across multiple sites can be leveraged to improve network performance.

2) Full trajectory learning

Instead of using a set of pre-defined functional forms for the (segmented) motion trajectories, a mapping
$\beta \rightarrow (\theta(t), p)$ is learned by the neural network. In other words, the network is trained to provide both a (full) motion trajectory as well as the associated probability. The motion estimation problem is then given by

$$\min_{\beta} \left\| AFST\big(\theta(\beta)\big)x - y \right\| + \lambda \log p(\beta)$$

i.e. the motion estimation is performed entirely in the network's latent space spanned by β.

3) Full trajectory parametrization

If a certain functional form can be assumed to directly approximate the motion trajectories sufficiently well, we have e.g. in the polynomial case:

$$\theta(t) = \sum_i \alpha_i t^i$$

Leading to the optimization problem

$$\min_{\alpha} \left\| AFST\big(\theta(\alpha)\big)x - y \right\| + \lambda \log p(\alpha)$$

i.e. the motion estimation is performed in the parameter space given by the above functional form.
In another embodiment the motion estimation and the reconstruction of the final motion-corrected magnetic resonance image are performed simultaneously as a generalized rigid motion-corrected or non-rigid motion-corrected reconstruction.
An advantage of the described medical system is that the computational burden is reduced. Typically, these optimization problems have been reserved for generalized rigid motion-corrected reconstructions. However, because the probability function greatly reduces or guides the optimization to particular solutions it now becomes feasible to also perform non-rigid motion-corrected reconstructions.
In another embodiment the calculated motion trajectory is formulated as a segmented and parameterized trajectory.
In another embodiment the motion trajectory is formulated as a polynomial.
In another embodiment the motion trajectory is formulated as a fully parameterized trajectory.
In another embodiment the motion trajectory is formulated as a series of harmonic functions.
In another embodiment the calculated motion trajectory is formulated as a time-dependent deformation vector field.
In another embodiment the motion estimating neural network is a sequence of fully connected layers.
In another embodiment the motion estimating neural network comprises multiple one-dimensional convolutional layers followed by at least one fully connected layer. The sequence of multiple fully connected layers or the multiple one-dimensional convolutional layer followed by at least one fully connected layer works well because these neural networks are very flexible and may enable accurate calculation of the trajectory probability.
In another embodiment the motion estimating neural network is further configured for outputting both the calculated motion trajectory and the trajectory probability in response to receiving an input vector. In this embodiment the motion estimating neural network provides not only the trajectory probability for the weighting function but also provides a calculated motion trajectory which can be used in the next iteration of the optimization problem. In certain instances, this may lead to an acceleration and even possibly more accurate determination of the subject's motion.
In another embodiment the motion estimating neural network is formed from multiple convolutional layers followed by an additional convolutional layer to output the calculated motion trajectory. The multiple convolutional layers are followed by at least one fully connected layer to output the trajectory probability.
In another embodiment the motion estimating neural network is formed from multiple convolutional layers followed by an additional convolutional layer to output the calculated motion trajectory. The multiple convolutional layers are followed by at least one pooling layer and at least one fully connected layer to output the trajectory probability.
In another embodiment the motion estimating neural network is formed from an input layer followed by multiple convolutional layers to output the calculated motion trajectory. The input layer is also connected to at least one fully connected layer to output the trajectory probability.
In another embodiment the trajectory data comprises a suggested motion trajectory in the predefined coordinate system. Modifying the optimization problem using the trajectory data comprises updating the trial motion trajectory to be a weighted sum of the modified motion trajectory and the trial motion trajectory.

Using the notation previously introduced, the optimization problem can be formulated as:

$$\min_{K} \frac{1}{2} \left\| AFST\big(\theta(K)\big)x - y \right\|_2^2$$

where in between iterations the vector K is updated by:

$$K = \alpha K + \beta N(K)$$

Where $\alpha$ and $\beta$ are weighting factors and $N(K)$ is suggested motion trajectory: the vector K input into the motion estimating neural network. In this equation the weighted sum of the trial motion trajectory and the suggested motion trajectory is performed.

In another embodiment the motion estimating neural network is a sequence of one-dimensional convolutional layers if the preferred coordinate system parameterizes rigid body motion of the subject and the motion estimating neural network outputs the suggested motion trajectory.

In another embodiment the motion estimating neural network is a sequence of fully connected layers if the preferred coordinate system parameterizes rigid body motion of the subject and the motion estimating neural network outputs the suggested motion trajectory.

In another embodiment the motion estimating neural network is a sequence of layers comprising both one dimensional convolutional layers and fully connected layers if the preferred coordinate system parameterizes rigid body motion of the subject and the motion estimating neural network outputs the suggested motion trajectory.

In another embodiment the motion estimating neural network is a sequence of three-dimensional convolutional layers if the preferred coordinate system parameterizes a deformation vector field and the motion estimating neural network outputs the suggested motion trajectory.

In another embodiment the motion estimating neural network is a sequence of two-dimensional convolutional layers for each slice of a three-dimensional volume if the preferred coordinate system parameterizes a deformation vector field and the motion estimating neural network outputs the suggested motion trajectory.

The above neural network architectures may be trained by using training data that comprises training trial motion data and training trajectory data. The training trajectory data comprises a training suggested motion trajectory. The training motion trajectory data can be obtained by solving the above optimization problem without updating the trial motion trajectory in between iterations. One can start with a trial motion trajectory and set this equal to being the training trial motion trajectory and then use the trajectory from the completed numerical optimization as the training suggested motion trajectory. After many pairs comprising of training trial motion data and training suggested motion trajectories have been collected the motion estimating neural network can be trained for example with a deep learning protocol with the training trial motion trajectory as input to the neural network and the training suggested motion trajectory as the ground truth data.

In another embodiment execution of the machine-executable instructions further causes the computational system to receive acquisition metadata descriptive of the measured k-space data and/or the subject. Execution of the machine-executable instructions further causes the computational system to select the motion estimating neural network from a database of motion estimating neural networks using the acquisition metadata. For a particular acquisition type or acquisition geometry the motion estimating neural network can be chosen. This very likely provides more accurate motion estimation.

In another embodiment the motion estimating neural network is further configured to receive the acquisition metadata as input. The step of inputting the trial motion trajectory into the motion estimating neural network would also comprise inputting at least a portion of the acquisition metadata into the motion estimating neural network. This may be beneficial because this may enable more detailed tailoring of the trajectory data for particular acquisition conditions, the position of the subject or even the age and gender of the subject.

In another embodiment execution of the machine-executable instructions further causes the computational system to receive training k-space data. Execution of the machine-executable instructions further causes the computational system to receive training trajectory probability data and preferably training motion trajectory data. For example, there may be some embodiments where there is only training trajectory probability data output and others where there is training motion trajectory data which is output also by the neural network. In some instances, the training k-space data may also include the acquisition metadata depending upon whether the neural network is trained to receive this also or not. Execution of the machine-executable instructions further causes the computational system to train the motion estimating neural network using the training k-space data and the training motion trajectory probability data and preferably the training motion trajectory data. In this case, the k-space data and possibly the acquisition metadata is known ahead of time as well as the trajectory probability data and possibly the training motion trajectory data. The training data can then be used to train the motion estimating neural network for example using a deep learning algorithm.

In another embodiment the motion estimating neural network is trained with a loss function that contains a function that is a derivative of the trial motion trajectory. Using a loss function that contains a function as a derivative of the trial motion trajectory may be beneficial because it may cause the output of the motion estimating neural network to be more stable.

In another embodiment the training trajectory probability data and/or preferably the training motion trajectory data is determined using any one of the following: a numerical solution of the optimization problem, a navigator, self-navigation data from the k-space data, optical data such as camera data, motion data from fiducial markers, and combinations thereof. This embodiment may be beneficial because measured motion of the subject may be used to assist in training the motion estimating neural network.

In another embodiment the medical system further comprises a magnetic resonance imaging system. The memory further stores pulse sequence commands configured to control the magnetic resonance imaging system to acquire the measured k-space data. Execution of the machine-executable instructions further causes the computational system to control the magnetic resonance imaging system with the pulse sequence commands to acquire the measured k-space data.

In another aspect the invention provides for a computer program comprising machine-executable instructions and a motion estimating neural network. The motion estimating neural network is configured for outputting trajectory data in response to receiving a trial motion trajectory as input. The trial motion trajectory has a predefined coordinate system. Execution of the machine-executable instructions causes the computational system to receive measured k-space data descriptive of a subject. The measured k-space data is divided into a sequence of discrete acquisitions. Execution of the machine-executable instructions further causes the computational system to perform motion estimation of the subject between the sequence of discrete acquisitions by iteratively solving an optimization problem to determine a calculated motion trajectory of the subject in the predefined coordinate system. The optimization problem is formulated to minimize a difference between the measured k-space data and a transformation of the re-sampled k-space data and a motion-corrected trial magnetic resonance image as a function of a trial motion trajectory and the measured k-space data.

Performing the motion estimation comprises receiving the trajectory data in response to inputting the trial motion trajectory into the motion estimating neural network. The optimization problem is then modified using the trajectory data. Execution of the machine-executable instructions further causes the computational system to reconstruct a final motion-corrected magnetic resonance image from the k-space data and the calculated motion trajectory in the predefined coordinate system.

In another aspect the invention provides for a method of medical imaging or a method of operating a medical system. The method comprises receiving measured k-space data descriptive of a subject. The measured k-space data is divided into a sequence of discrete acquisitions. The method further comprises performing motion estimation of the subject between the sequence of discrete acquisitions by solving an optimization problem to determine a calculated motion trajectory of the subject in the preferred coordinate system. The optimization problem is formulated to minimize the difference between the measured k-space data and a transformation of re-sampled k-space data of a motion-corrected trial magnetic resonance image as a function of a trial motion trajectory and the measured k-space data.

Performing the motion estimation comprises receiving the trajectory data in response to inputting the trial motion trajectory into a motion estimating neural network. The trial motion trajectory has a predefined coordinate system. The optimization problem then modified using the trajectory data. The method further comprises reconstructing a final motion-corrected magnetic resonance image from the k-space data and the calculated motion trajectory in the predefined coordinate system.

It is understood that one or more of the aforementioned embodiments of the invention may be combined as long as the combined embodiments are not mutually exclusive.

As will be appreciated by one skilled in the art, aspects of the present invention may be embodied as an apparatus, method or computer program product. Accordingly, aspects of the present invention may take the form of an entirely hardware embodiment, an entirely software embodiment (including firmware, resident software, micro-code, etc.) or an embodiment combining software and hardware aspects that may all generally be referred to herein as a "circuit," "module" or "system." Furthermore, aspects of the present invention may take the form of a computer program product embodied in one or more computer readable medium(s) having computer executable code embodied thereon.

Any combination of one or more computer readable medium(s) may be utilized. The computer readable medium may be a computer readable signal medium or a computer readable storage medium. A 'computer-readable storage medium' as used herein encompasses any tangible storage medium which may store instructions which are executable by a processor or computational system of a computing device. The computer-readable storage medium may be referred to as a computer-readable non-transitory storage medium. The computer-readable storage medium may also be referred to as a tangible computer readable medium. In some embodiments, a computer-readable storage medium may also be able to store data which is able to be accessed by the computational system of the computing device. Examples of computer-readable storage media include, but are not limited to: a floppy disk, a magnetic hard disk drive, a solid state hard disk, flash memory, a USB thumb drive, Random Access Memory (RAM), Read Only Memory (ROM), an optical disk, a magneto-optical disk, and the register file of the computational system. Examples of optical disks include Compact Disks (CD) and Digital Versatile Disks (DVD), for example CD-ROM, CD-RW, CD-R, DVD-ROM, DVD-RW, or DVD-R disks. The term computer readable-storage medium also refers to various types of recording media capable of being accessed by the computer device via a network or communication link. For example, data may be retrieved over a modem, over the internet, or over a local area network. Computer executable code embodied on a computer readable medium may be transmitted using any appropriate medium, including but not limited to wireless, wire line, optical fiber cable, RF, etc., or any suitable combination of the foregoing.

A computer readable signal medium may include a propagated data signal with computer executable code embodied therein, for example, in baseband or as part of a carrier wave. Such a propagated signal may take any of a variety of forms, including, but not limited to, electro-magnetic, optical, or any suitable combination thereof. A computer readable signal medium may be any computer readable medium that is not a computer readable storage medium and that can communicate, propagate, or transport a program for use by or in connection with an instruction execution system, apparatus, or device.

'Computer memory' or 'memory' is an example of a computer-readable storage medium. Computer memory is any memory which is directly accessible to a computational system. 'Computer storage' or 'storage' is a further example of a computer-readable storage medium. Computer storage is any non-volatile computer-readable storage medium. In some embodiments computer storage may also be computer memory or vice versa.

A 'computational system' as used herein encompasses an electronic component which is able to execute a program or machine executable instruction or computer executable code. References to the computational system comprising the example of "a computational system" should be interpreted as possibly containing more than one computational system or processing core. The computational system may for instance be a multi-core processor. A computational system may also refer to a collection of computational systems within a single computer system or distributed amongst multiple computer systems. The term computational system should also be interpreted to possibly refer to a collection or network of computing devices each comprising a processor or computational systems. The machine executable code or instructions may be executed by multiple computational systems or processors that may be within the same computing device or which may even be distributed across multiple computing devices.

Machine executable instructions or computer executable code may comprise instructions or a program which causes a processor or other computational system to perform an aspect of the present invention. Computer executable code for carrying out operations for aspects of the present invention may be written in any combination of one or more programming languages, including an object-oriented programming language such as Java, Smalltalk, C++ or the like and conventional procedural programming languages, such as the "C" programming language or similar programming languages and compiled into machine executable instructions. In some instances, the computer executable code may be in the form of a high-level language or in a pre-compiled form and be used in conjunction with an interpreter which generates the machine executable instructions on the fly. In other instances, the machine executable instructions or computer executable code may be in the form of programming for programmable logic gate arrays.

The computer executable code may execute entirely on the user's computer, partly on the user's computer, as a stand-alone software package, partly on the user's computer and partly on a remote computer or entirely on the remote computer or server. In the latter scenario, the remote computer may be connected to the user's computer through any type of network, including a local area network (LAN) or a wide area network (WAN), or the connection may be made to an external computer (for example, through the Internet using an Internet Service Provider).

Aspects of the present invention are described with reference to flowchart illustrations and/or block diagrams of methods, apparatus (systems) and computer program products according to embodiments of the invention. It is understood that each block or a portion of the blocks of the flowchart, illustrations, and/or block diagrams, can be implemented by computer program instructions in form of computer executable code when applicable. It is further understood that, when not mutually exclusive, combinations of blocks in different flowcharts, illustrations, and/or block diagrams may be combined. These computer program instructions may be provided to a computational system of a general purpose computer, special purpose computer, or other programmable data processing apparatus to produce a machine, such that the instructions, which execute via the computational system of the computer or other programmable data processing apparatus, create means for implementing the functions/acts specified in the flowchart and/or block diagram block or blocks.

These machine executable instructions or computer program instructions may also be stored in a computer readable medium that can direct a computer, other programmable data processing apparatus, or other devices to function in a particular manner, such that the instructions stored in the computer readable medium produce an article of manufacture including instructions which implement the function/act specified in the flowchart and/or block diagram block or blocks.

The machine executable instructions or computer program instructions may also be loaded onto a computer, other programmable data processing apparatus, or other devices to cause a series of operational steps to be performed on the computer, other programmable apparatus or other devices to produce a computer implemented process such that the instructions which execute on the computer or other programmable apparatus provide processes for implementing the functions/acts specified in the flowchart and/or block diagram block or blocks.

A 'user interface' as used herein is an interface which allows a user or operator to interact with a computer or computer system. A 'user interface' may also be referred to as a 'human interface device.' A user interface may provide information or data to the operator and/or receive information or data from the operator. A user interface may enable input from an operator to be received by the computer and may provide output to the user from the computer. In other words, the user interface may allow an operator to control or manipulate a computer and the interface may allow the computer to indicate the effects of the operator's control or manipulation. The display of data or information on a display or a graphical user interface is an example of providing information to an operator. The receiving of data through a keyboard, mouse, trackball, touchpad, pointing stick, graphics tablet, joystick, gamepad, webcam, headset, pedals, wired glove, remote control, and accelerometer are all examples of user interface components which enable the receiving of information or data from an operator.

A 'hardware interface' as used herein encompasses an interface which enables the computational system of a computer system to interact with and/or control an external computing device and/or apparatus. A hardware interface may allow a computational system to send control signals or instructions to an external computing device and/or apparatus. A hardware interface may also enable a computational system to exchange data with an external computing device and/or apparatus. Examples of a hardware interface include, but are not limited to: a universal serial bus, IEEE 1394 port, parallel port, IEEE 1284 port, serial port, RS-232 port, IEEE-488 port, Bluetooth connection, Wireless local area network connection, TCP/IP connection, Ethernet connection, control voltage interface, MIDI interface, analog input interface, and digital input interface.

A 'display' or 'display device' as used herein encompasses an output device or a user interface adapted for displaying images or data. A display may output visual, audio, and or tactile data. Examples of a display include, but are not limited to: a computer monitor, a television screen, a touch screen, tactile electronic display, Braille screen,

Cathode ray tube (CRT), Storage tube, Bi-stable display, Electronic paper, Vector display, Flat panel display, Vacuum fluorescent display (VF), Light-emitting diode (LED) displays, Electroluminescent display (ELD), Plasma display panels (PDP), Liquid crystal display (LCD), Organic light-emitting diode displays (OLED), a projector, and Head-mounted display.

Medical imaging data is defined herein as being recorded measurements made by a tomographic medical imaging system descriptive of a subject. The medical imaging data may be reconstructed into a medical image. A medical image id defined herein as being the reconstructed two- or three-dimensional visualization of anatomic data contained within the medical imaging data. This visualization can be performed using a computer.

K-space data is defined herein as being the recorded measurements of radio frequency signals emitted by atomic spins using the antenna of a Magnetic resonance apparatus during a magnetic resonance imaging scan. Magnetic resonance data is an example of tomographic medical image data.

A Magnetic Resonance Imaging (MRI) image or MR image is defined herein as being the reconstructed two- or three-dimensional visualization of anatomic data contained within the magnetic resonance imaging data. This visualization can be performed using a computer.

BRIEF DESCRIPTION OF THE DRAWINGS

In the following preferred embodiments of the invention will be described, by way of example only, and with reference to the drawings in which:

Fig. 1 illustrates an example of a medical system;
Fig. 2 shows a flow chart which illustrates a method of using the medical system of Fig. 1;
Fig. 3 illustrates an example of a medical system;
Fig. 4 shows a flow chart which illustrates a method of using the medical system of Fig. 3;
Fig. 5 shows a flow chart which illustrates a method of training a motion estimating neural network;
Fig. 6 illustrates an implementation of a medical system;
Fig. 7 illustrates an implementation of a motion estimating neural network;
Fig. 8 illustrates a further implementation of a motion estimating neural network;
Fig. 9 illustrates a further implementation of a motion estimating neural network; and
Fig. 10 illustrates a further implementation of a motion estimating neural network.

DETAILED DESCRIPTION OF EMBODIMENTS

Like numbered elements in these figures are either equivalent elements or perform the same function. Elements which have been discussed previously will not necessarily be discussed in later figures if the function is equivalent.

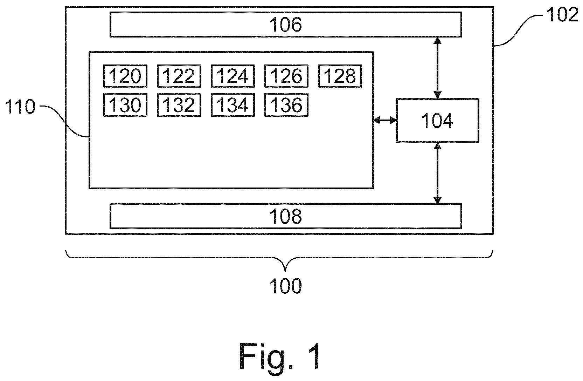

Fig. 1 illustrates an example of a medical system 100. The medical system 100 is shown as comprising a computer 102. The computer 102 is intended to represent one or more computers or computing systems at one or more locations. The computer 102 is shown as comprising a computational system 104. The computational system 104 could represent one or more computational systems or computing cores at one or more locations also. The computational system 104 is shown as being in communication with an optional hardware interface 106. The optional hardware interface 106 may enable the computational system 104 to control and operate other components such as a magnetic resonance imaging system. The computational system 104 is further shown as being in communication with an optional user interface 108 that may enable a user to operate and/or control the medical system 100. The computational system 104 is further shown as being in communication with a memory 110. The memory 110 is intended to represent various types of memory that may be accessible to the computational system 104. For example, the memory 110 may represent a non-transitory storage medium.

The memory 110 is shown as containing machine-executable instructions 120. The machine-executable instructions 120 enable the computational system 104 to perform basic tasks such as computation, image processing, and control of other components if present. The memory 110 is further shown as containing a motion estimating neural network 122. The memory 110 is further shown as containing measured k-space data 124. The measured k-space data 124 may be descriptive of a subject and is divided into a sequence of discrete acquisitions or shots. The memory 110 is further shown as containing an optimization module 126 that is used to solve for a calculated motion trajectory 134.

The optimization module 126 varies a trial motion trajectory 128 and looks at the resulting motion-corrected trial magnetic resonance image 132. The optimization module 126 uses the motion estimating neural network 122 and may have a cost function which contains a function dependent upon a trajectory probability that is output by the motion estimating neural network 122 in response to receiving a trial motion trajectory 128. The memory 110 is further shown as containing a calculated motion trajectory 134 that was solved for by the optimization module 126. The memory 110 is further shown as containing a final motion-corrected magnetic resonance image 136 that was reconstructed using the measured k-space data 124 and the calculated motion trajectory 134.

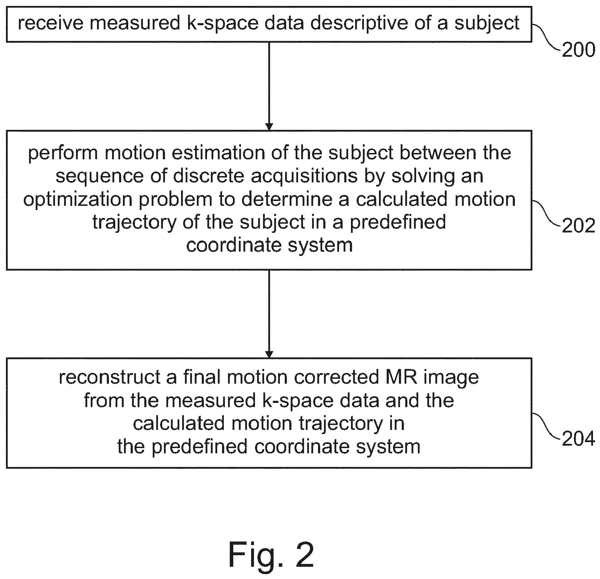

Fig. 2 shows a flowchart which illustrates a method of operating the medical system 100 of Fig. 1. First, in step 200, the measured k-space data 124 is received. As was mentioned previously, the measured k-space data 124 is descriptive of the subject and is divided into a sequence of discrete acquisitions. Next, in step 202, motion estimation of the subject is performed to determine motion between the sequence of discrete acquisitions by solving an optimization problem with the optimization module 126 to determine the calculated motion trajectory 134 of the subject in a predefined coordinate system. The optimization problem is formulated to minimize a difference between the measured k-space data 124 and a transformation of re-sampled k-space data of a motion-corrected trial magnetic resonance image as a function of a trial motion trajectory and the measured k-space data.

Performing the motion estimation comprises receiving the trajectory data 130 in response to inputting the trial motion trajectory 128 into the motion estimating neural network 122. The optimization problem comprises a cost function that is a function of the trajectory data (the trajectory probability) or an intermediate step between interactions that updates or modifies the trial motion trajectory with the trajectory data (suggested motion trajectory). Finally, in step 204, the final motion-corrected magnetic resonance image 136 is reconstructed from the k-space data 124 and the calculated motion trajectory 134.

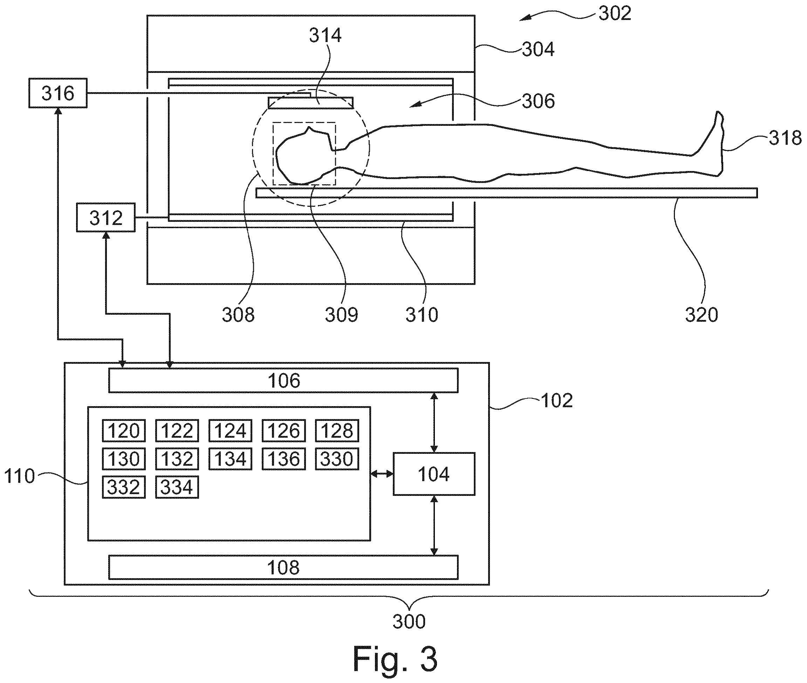

Fig. 3 illustrates a further example of a medical system 300. The medical system 300 depicted in Fig. 3 is similar to the medical system 100 in Fig. 1 except that it additionally comprises a magnetic resonance imaging system 302 that is controlled by the computational system 104.

The magnetic resonance imaging system 302 comprises a magnet 304. The magnet 304 is a superconducting cylindrical type magnet with a bore 306 through it. The use of different types of magnets is also possible; for instance it is also possible to use both a split cylindrical magnet and a so called open magnet. A split cylindrical magnet is similar to a standard cylindrical magnet, except that the cryostat has been split into two sections to allow access to the iso-plane of the magnet, such magnets may for instance be used in conjunction with charged particle beam therapy. An open magnet has two magnet sections, one above the other with a space in-between that is large enough to receive a subject: the arrangement of the two sections area similar to that of a Helmholtz coil. Open magnets are popular, because the subject is less confined. Inside the cryostat of the cylindrical magnet there is a collection of superconducting coils.

Within the bore 306 of the cylindrical magnet 304 there is an imaging zone 308 where the magnetic field is strong and uniform enough to perform magnetic resonance imaging. A field of view 309 is shown within the imaging zone 308. The k-space data that is acquired typically acquried for the field of view 309. The region of interest could be identical with the field of view 309 or it could be a sub volume of the field of view 309. A subject 318 is shown as being supported by a subject support 320 such that at least a portion of the subject 318 is within the imaging zone 308 and the field of view 309.

Within the bore 306 of the magnet there is also a set of magnetic field gradient coils 310 which is used for acquisition of preliminary k-space data to spatially encode magnetic spins within the imaging zone 308 of the magnet 304. The magnetic field gradient coils 310 connected to a magnetic field gradient coil power supply 312. The magnetic field gradient coils 310 are intended to be representative. Typically magnetic field gradient coils 310 contain three separate sets of coils for spatially encoding in three orthogonal spatial directions. A magnetic field gradient power supply supplies current to the magnetic field gradient coils. The current supplied to the magnetic field gradient coils 310 is controlled as a function of time and may be ramped or pulsed.

Adjacent to the imaging zone 308 is a radio-frequency coil 314 for manipulating the orientations of magnetic spins within the imaging zone 308 and for receiving radio transmissions from spins also within the imaging zone 308. The radio frequency antenna may contain multiple coil elements. The radio frequency antenna may also be referred to as a channel or antenna. The radio-frequency coil 314 is connected to a radio frequency transceiver 316. The radio-frequency coil 314 and radio frequency transceiver 316 may be replaced by separate transmit and receive coils and a separate transmitter and receiver. It is understood that the radio-frequency coil 314 and the radio frequency transceiver 316 are representative. The radio-frequency coil 314 is intended to also represent a dedicated transmit antenna and a dedicated receive antenna. Likewise the transceiver 316 may also represent a separate transmitter and receivers. The radio-frequency coil 314 may also have multiple receive/transmit elements and the radio frequency transceiver 316 may have multiple receive/transmit channels. The transceiver 316 and the gradient controller 312 are shown as being connected to the hardware interface 106 of the computer system 102.

The memory 110 is further shown as containing pulse sequence commands 330. The pulse sequence commands are commands or data which may be converted into commands which can control the magnetic resonance imaging system 302 to acquire the measured k-space data 124. The memory 110 is further shown as containing acquisition metadata 332. This may be details taken from the pulse sequence commands 330, as well as data entered by an operator describing the setup of the magnetic resonance imaging system 302 and/or the details about the subject 318. The acquisition metadata 332 may also contain information about the anatomical region of the subject 318 imaged. The memory 110 is further shown as optionally containing a database of motion estimating neural networks 334. For example, there may be different motion estimating neural networks for different anatomical regions of the subject. The acquisition metadata 332 or a portion of the acquisition data 332 may be used to query the database 334 to retrieve the motion estimating neural network 122.

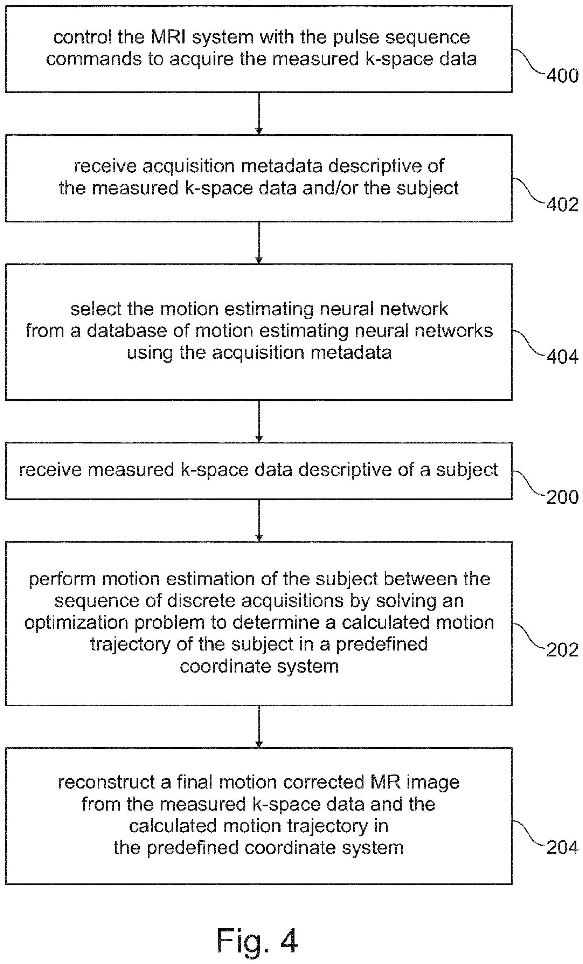

Fig. 4 shows a flow chart which illustrates a method of operating the medical system 300 of Fig. 3. The method starts at step 400 where the magnetic resonance imaging system 300 is controlled with the pulse sequence commands 330 to acquire the measured k-space data 124. Next in step 402, acquisition metadata 332 descriptive of the measured k-space data and/or the subject is received. The acquisition metadata 332 could be generated automatically from data from the pulse sequence commands 330 and/or it could be generated from data entered by an operator. The motion estimating neural network 122 may be further configured to receive the acquisition metadata as input. Alternatively, or in addition to this, the acquisition metadata 332 or a portion of the acquisition metadata is used to query the database of motion estimating neural networks to obtain the motion estimating neural network 122. After step 402, the method proceeds with steps 200, 202, and 204 as was illustrated in Fig. 2.

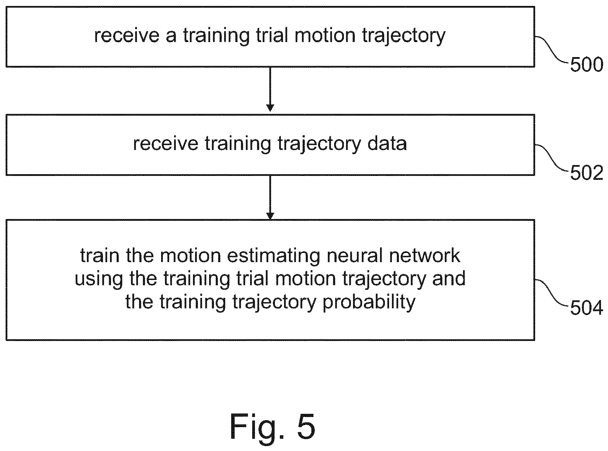

Fig. 5 shows a flowchart which illustrates a method of training the motion estimating neural network. First, in step 500, training trial motion trajectory is received. Next, in step 502, training trajectory data is received. The combination of the training trial motion trajectory and the training trajectory data represents the data which may be used to train the motion estimating neural network. For example, the training trial motion trajectory is input into the motion estimating neural network and the output is compared to the associated training trajectory data. In step 504, the motion estimating neural network is trained using the training trial motion trajectory as the input to the neural network and the training trajectory probability is used as the ground truth data. This may for example be done using a deep learning training algorithm. The training process may also be done as a vector or parallel process.

In examples, a motion estimating neural network 122 is used to predict the probability distribution (trajectory probability 130) of patient motion trajectories for a given scan type. The network receives as input metadata that may affect the resulting distribution, both about the patient as well as about the MR sequence. The network-predicted probability distribution is used to guide a motion-compensated reconstruction algorithm by constraining the search space to high-probability regions, leading to improved convergence characteristics and reduced computation times. Training of the network is realized using ground truth motion trajectories that are obtained either using external sensors, e.g. in-bore cameras or by (unguided) motion-compensated reconstructions.

Image degradation due to subject motion during the acquisition is a persistent problem in the clinical application of magnetic resonance imaging (MRI). The associated artifacts typically appear as ghosting or blurring in the images and often reduce image quality to a degree that makes medical analysis impossible.

Due to the clinical relevance of motion artifacts, many solutions have been proposed by the MR research community. In particular, motion-compensated reconstruction methods have been shown to allow for substantial reduction of motion artifact levels in many cases.

Motion-compensated reconstruction methods attempt to estimate the exact motion parameters as a function of scan time. Even for simple rigid motion models as commonly employed for neuro scans, however, the resulting optimization problem is typically high-dimensional and non-convex. Consequently, long computation times are often required to obtain a motion-compensated result, reaching up to several hours for severe motion cases, even if computations are performed on a GPU. In addition, it is possible that the algorithm gets "stuck" in a local minimum, i.e. a sub-optimal result with residual motion artifacts is obtained.

Examples may overcome these limitations by reducing the solution space of the motion parameter estimation problem. Based on the assumption that the space of realistic patient motion trajectories has a much lower dimension than the space of all possible motion trajectories, a learningbased approach is described to infer corresponding constraints from clinical motion-corrupted patient data.

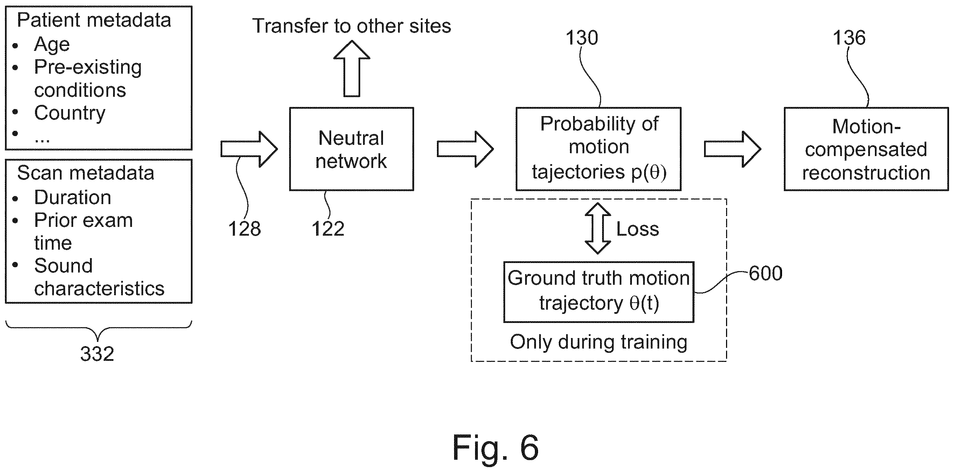

A general overview of some examples is shown below in Fig. 6. A neural network (motion estimating neural network 122) is used to predict the probability distribution (trajectory probability 130) of patient motion trajectories for a given scan type (without loss of generality, only a single MR sequence of fixed length is assumed in the following). The network may receive as input metadata that may affect this probability distribution, both about the patient - such as age, previous medical diagnoses, etc. - as well as about the MR sequence - e.g. expected scan time and sound characteristics. Training of the network is realized using ground truth motion trajectories that are obtained either using external sensors, e.g. in-bore cameras or by (potentially time-consuming) motion-compensated reconstructions. In the latter case, successful convergence of the algorithm is confirmed to ensure correctness of the estimated trajectories, e.g. by visual inspection.

During inference, the network-predicted probability distribution is used to guide a motion-compensated reconstruction algorithm by constraining the search space to high-probability regions. Importantly, the entire system can be trained after deployment, leading to increasingly faster and more robust reconstructions due to continuously refined probability predictions as more training data becomes available.

Fig. 6 illustrates an implementation of a medical system. The motion estimating neural network 122 is shown as receiving as input acquisition metadata 332 and the trial motion trajectory 128; in response it outputs the trajectory probability 130. The acquisition metadata 332 is shown as comprising either patient metadata and/or scan metadata. The trajectory probability 130 is used with the measured k-space data 124 to calculate the final motion-corrected magnetic resonance image 136. The system may additionally be trained by comparing the trajectory probability 130 with training motion trajectory data 600. For example, it could be done during deployment of the medical system or it could be done beforehand and the neural network 122 could be transferred or be used as different sites.

Additional examples may contain one or more of the following features:
Importantly, additional input data can be used to enable the neural network to produce an even further refined predicted motion trajectory subspace. In one embodiment, the network also receives the corrupted k-space data as input, allowing for a rough estimate of the realistic patient motion given the acquired data.

Learned probabilities can transferred from one MR sequence to another, given that the relevant metadata is similar (duration, sound characteristics, etc.).

Additional constraints on the mapping learned by the neural network are introduced during training to improve convergence characteristics of the resulting motion estimation problem. As an example, for the second embodiment given above ("full trajectory learning"), a term $\gamma \left\| \frac{\partial \theta}{\partial \beta} \right\|$ and/or $\gamma \left\| \frac{\partial p}{\partial \beta} \right\|$ can be added to the loss function during network training to enforce smoothness of the learned mapping $\beta \rightarrow (\theta(t), p)$. The regularization parameter $\gamma$ defines the resulting "smoothness" of the mapping. Adding such constraints can help avoid local minima during the motion estimation.

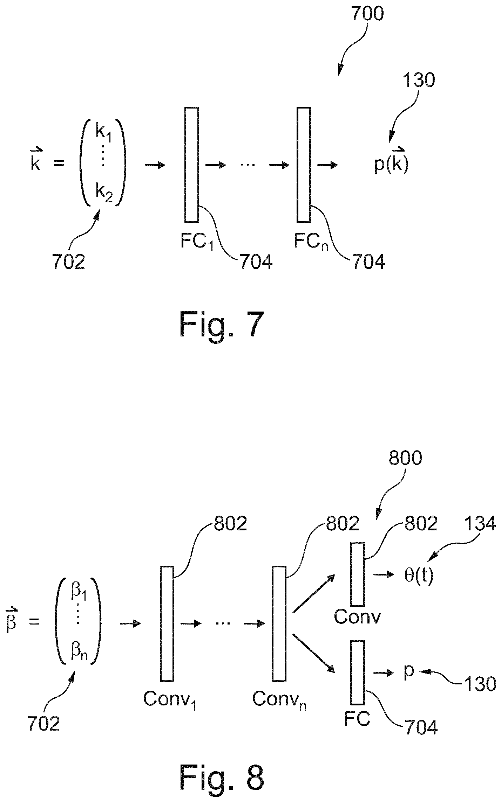

Fig. 7 illustrates an example of an implementation of a motion estimating neural network 700. There is an input vector 702 that is fed into a series of fully connected layers 704. The final fully connected layer outputs the trajectory data 130. The trajectory data may be either a trajectory probability and/or a suggested motion trajectory. The input vector 702 may be the trial motion trajectory 128 or a combination of both the trial motion trajectory 128 and the position metadata 332. The neural network structure illustrated in Fig. 7 may be referred to as a multilayer perceptron.

Fig. 8 illustrates a further example of an implementation of a motion estimating neural network 800. The input vector 702 is input into a first convolutional layer 802. This is then put through a series of n convolutional layers 802. After this sequence at least one additional convolutional layer 800 forms one branch and at least one fully connected layer 704 forms a second branch. The convolutional layers 800 output a calculated or predicted motion trajectory 134 or a suggested motion trajectory. The fully connected layer 704 outputs the trajectory probability 130. If it were desired to form a neural network 800 that did not output the calculated motion trajectory 134 this branch with the convolutional layer 800 and the outputted calculated motion trajectory 134 could simply be removed and deleted. The architecture would then be a series of convolutional layers 802 followed by a fully connected layer 704.

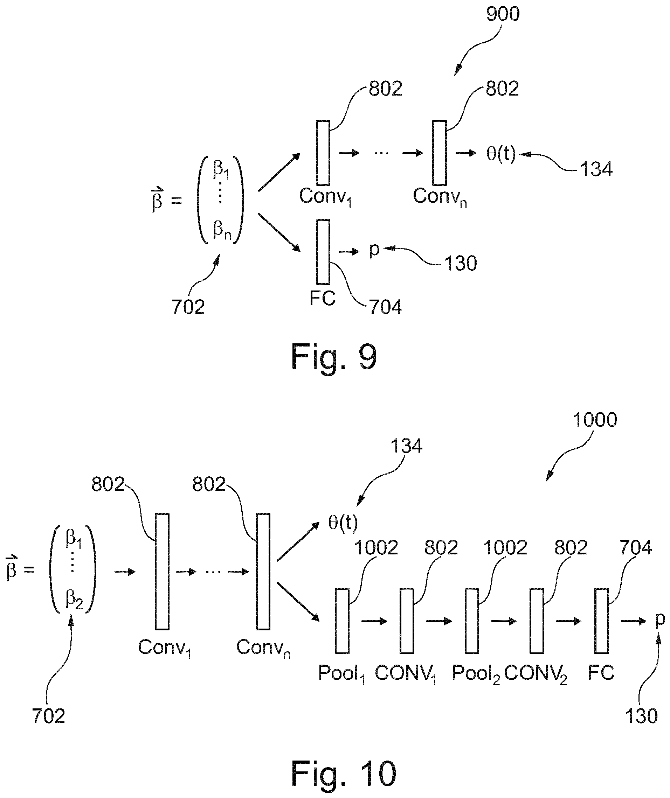

Fig. 9 illustrates a further example of an implementation of the motion estimating neural network 900. The input vector 702 is fed into two separate branches. One branch goes into a series of convolutional layers 802 which then output the calculated motion trajectory 134 or a suggested motion trajectory. The input vector 702 is also separately fed to at least one or a series of fully connected layers 704, which then output the trajectory probability 130. It is noted that if the branch which outputs the calculated motion trajectory 134 is deleted, then the neural network 900 reverts to what is illustrated in Fig. 7 for the network 700.

Fig. 10 illustrates a further implementation of a motion estimating neural network 1000. The input vector 702 is first fed into a series of convolutional layers 802. The output of the convolutional layers may for example go to an output layer which then outputs the calculated motion trajectory 134 or a suggested motion trajectory. The output of the series of convolutional layers 802 may then also be fed into another branch which first goes into a pooling or down-sampling layer 102, into a convolutional layer 802, which is then fed into an additional pooling layer 102, which is then fed again into another convolutional layer 802, and finally a fully connected layer 704. This then outputs the trajectory probability 130.

While the invention has been illustrated and described in detail in the drawings and foregoing description, such illustration and description are to be considered illustrative or exemplary and not restrictive; the invention is not limited to the disclosed embodiments.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measured cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

## REFERENCE SIGNS LIST

100 medical system
102 computer
104 computational system
106 hardware interface
108 user interface
110 memory
120 machine executable instructions
122 motion estimating neural network
124 measured k-space data
126 optimization module
128 trial motion trajectory
130 trajectory probability
132 motion-corrected trial magnetic resonance image
134 calculated motion trajectory
136 final motion corrected magnetic resonance image
200 receive measured k-space data descriptive of a subject
202 perform motion estimation of the subject between the sequence of discrete acquisitions by solving an optimization problem to determine a calculated motion trajectory of the subject in the predefined coordinate system
204 reconstruct a final motion corrected magnetic resonance image from the measured k-space data and the calculated motion trajectory in the predefined coordinate system
300 medical system
302 magnetic resonance imaging system
304 magnet
306 bore of magnet
308 imaging zone
309 field of view
310 magnetic field gradient coils
312 magnetic field gradient coil power supply
314 radio-frequency coil
316 transceiver
318 subject
320 subject support
330 pulse sequence commands
332 acquisition metadata
334 database of motion estimating neural networks
400 control the magnetic resonance imaging system with the pulse sequence commands to acquire the measured k-space data
402 receive acquisition metadata descriptive of the measured k-space data and/or the subject, wherein the motion estimating neural network is further configured to receive the acquisition metadata as input
404 select the motion estimating neural network from a database of motion estimating neural networks using the acquisition metadata
500 receive a training trial motion trajectory
502 receive a training trajectory probability and preferably training calculated motion trajectory
504 train the motion estimating neural network using the training trial motion trajectory and the training trajectory probability and preferably the training calculated motion trajectory
600 training motion trajectory data
700 implementation of motion estimating neural network
702 input vector
704 fully connected layer
800 implementation of motion estimating neural network
802 convolutional layer
900 implementation of motion estimating neural network
902 pooling (down sampling) layer
1000 implementation of motion estimating neural network

**Claims**

1. A medical system (100, 300) comprising:

   - a memory (110) storing machine executable instructions (120) and a motion estimating neural network (122, 700, 800, 900, 1000), wherein the motion estimating neural network is configured for outputting trajectory data (130) in response to receiving a trial motion trajectory (128) as input, wherein the trial motion trajectory has a predefined coordinate system;
   - a computational system (104), wherein execution of the machine executable instructions causes the computational system to:

      - receive (200) measured k-space data (124) descriptive of a subject (318), wherein the measured k-space data is divided into a sequence of discrete acquisitions;
      - perform (202) motion estimation of the subject between the sequence of discrete acquisitions by solving an optimization problem to determine a calculated motion trajectory (134) of the subject in the predefined coordinate system, wherein the optimization problem is formulated to iteratively minimize a difference between the measured k-space data and a transformation of resampled k-space data of a motion-corrected trial magnetic resonance image as a function of a trial motion trajectory and the measured k-space data, wherein performing the motion estimation comprises receiving the trajectory data in response to inputting the trial motion trajectory into the motion estimating neural network, wherein performing the motion estimation further comprises modifying optimization problem using the trajectory data; and
      - reconstruct (204) a final motion corrected magnetic resonance image (136) from the measured k-space data and the calculated motion trajectory in the predefined coordinate system.

2. The medical system of claim 1, wherein the calculated motion trajectory is formulated as any one of the following: a segmented and parameterized trajectory, a polynomial, a fully parameterized trajectory, as a deformation vector field, and a series of harmonic functions.

3. The medical system of claim 1 or 2, wherein the trajectory data comprises trajectory probability data, wherein the optimization problem comprises a cost function that is a function of the trajectory probability.

4. The medical system of claim 2 or 3, wherein the motion estimating neural network is any one of the following:

   - a sequence (600) of multiple fully connected layers (704); and
   - multiple (800, 1000) one-dimensional convolutional layers (802) followed by at least one fully connected layer (704).

5. The medical system of claim 1 or 2, wherein the motion estimating neural network is further configured for outputting both the calculated motion trajectory and the trajectory probability in response to receiving the trial motion trajectory.

6. The medical system of claim 5, wherein the trial motion trajectory spans a latent space of the motion estimation neural network.

7. The medical system of claim 5 or 6, wherein the motion estimating neural network is any one of the following:

   - multiple (800) convolutional layers (802) followed by an additional convolutional layer (802) to output the calculated motion trajectory (134), wherein the multiple convolutional layers are followed by at least one fully connected layer (704) to output the trajectory probability (130); and
   - multiple (1000) convolutional layers (802) followed by an additional convolutional layer (802) to output the calculated motion trajectory (134), wherein the multiple convolutional layers are followed by at least one pooling layer (1002) and at least one fully connected layer (704) to output the trajectory probability; and
   - an (900) input layer that is followed by multiple convolutional layers (802) to output the calculated motion trajectory (134), wherein the input layer is further connected to at least one fully connected layer (704) to output the trajectory probability.

8. The medical system of any one of claims 1 through 6, wherein the trajectory data comprises a suggested motion trajectory in the predefined coordinate system, wherein modifying optimization problem using the trajectory data comprises updating the trial motion trajectory to be a weighted sum of the suggested motion trajectory and the trial motion trajectory.

9. The medical system of claim 8, wherein the motion estimating neural network is any one of the following:

- a sequence of one-dimensional convolutional layers if the preferred coordinate system parameterizes rigid body motion of the subject;
- a sequence (700) of fully connected layers if the preferred coordinate system parameterizes rigid body motion of the subject;
- a sequence of layers comprising both one dimensional convolutional layers and fully connected layers if the preferred coordinate system parameterizes rigid body motion of the subject;
- a sequence of three-dimensional convolutional layers if the preferred coordinate system parameterizes a deformation vector field; and
- a sequence of two-dimensional convolutional layers for each slice of a three-dimensional volume if the preferred coordinate system parameterizes a deformation vector field.

10. The medical system of any one of the preceding claims, wherein execution of the machine executable instructions further causes the computational system to receive (402) acquisition metadata (332) descriptive of the measured k-space data and/or the subject, wherein the motion estimating neural network is further configured to receive the acquisition metadata as input.

11. The medical system of claim 10, wherein execution of the machine executable instructions further causes the computational system to select (404) the motion estimating neural network from a database of motion estimating neural networks (334) using the acquisition metadata.

12. The medical system of any one of the preceding claims, wherein execution of the machine executable instructions further causes the computational system to:

- receive (500) a training trial motion trajectory (600);
- receive (502) training trajectory data;
- train (504) the motion estimating neural network using the training trial motion trajectory and the training trajectory data, , wherein the wherein the motion estimating neural network is preferably trained with a loss function that contains a function that is a derivative of the trial motion trajectory

13. The medical system of any one of the preceding claims, wherein the medical system further comprises a magnetic resonance imaging system (302), wherein the memory further stores pulse sequence commands (330) configured to control the magnetic resonance imaging system to acquire the measured k-space data, wherein execution of the machine executable instructions further causes the computational system to control (400) the magnetic resonance imaging system with the pulse sequence commands to acquire the measured k-space data.

14. A computer program comprising machine executable instructions (120) and a motion estimating neural network (122, 700, 800, 900, 1000), wherein the motion estimating neural network is configured for outputting trajectory data (130) in response to receiving a trial motion trajectory (128) as input, wherein the trial motion trajectory has a predefined coordinate system, wherein execution of the machine executable instructions causes the computational system to:

- receive (200) measured k-space data (124) descriptive of a subject (318), wherein the measured k-space data is divided into a sequence of discrete acquisitions;
- perform (202) motion estimation of the subject between the sequence of discrete acquisitions by solving an optimization problem to determine a calculated motion trajectory (134) of the subject in the predefined coordinate system, wherein the optimization problem is formulated to iteratively minimize a difference between the measured k-space data and a transformation of resampled k-space data of a motion-corrected trial magnetic resonance image as a function of a trial motion trajectory and the measured k-space data, wherein performing the motion estimation comprises receiving the trajectory data in response to inputting the trial motion trajectory into the motion estimating neural network, wherein modifying the optimization problem further comprises modifying the optimization problem using the trajectory data; and
- reconstruct (204) a final motion corrected magnetic resonance image (136) from measured the k-space data and the calculated motion trajectory in the predefined coordinate system.

15. A method of medical imaging, wherein the method comprises:

- receiving (200) measured k-space data (124) descriptive of a subject (318), wherein the measured k-space data is divided into a sequence of discrete acquisitions;
- performing (202) motion estimation of the subject between the sequence of discrete acquisitions by solving an optimization problem to determine a calculated motion trajectory (134) of the subject in the predefined coordinate system, wherein the optimization problem is formulated to minimize a difference between the measured k-space data and a transformation of resampled k-space data of a motion-corrected trial magnetic resonance image as a function of a trial motion trajectory and the measured k-space data, wherein performing the motion estimation comprises receiving trajectory data in response to inputting a trial motion trajectory (128) into a motion estimating neural network (122, 700, 800, 900, 1000), wherein the motion estimating neural network is configured for the trajectory data in response to receiving the trial motion trajectory as input, wherein the trial motion trajectory has a predefined coordinate system, wherein performing the motion estimation further comprises modifying the optimization problem using the trajectory data; and

- reconstructing (204) a final motion corrected magnetic resonance image (136) from the measured k-space data and the calculated motion trajectory in the predefined coordinate system.

Fig. 1

receive measured k-space data descriptive of a subject

200

perform motion estimation of the subject between the sequence of discrete acquisitions by solving an optimization problem to determine a calculated motion trajectory of the subject in a predefined coordinate system

202

reconstruct a final motion corrected MR image from the measured k-space data and the calculated motion trajectory in the predefined coordinate system

204

Fig. 2

Fig. 3

control the MRI system with the pulse sequence
commands to acquire the measured k-space data — 400

receive acquisition metadata descriptive of
the measured k-space data and/or the subject — 402

select the motion estimating neural network
from a database of motion estimating neural networks
using the acquisition metadata — 404

receive measured k-space data descriptive of a subject — 200

perform motion estimation of the subject between the
sequence of discrete acquisitions by solving an
optimization problem to determine a calculated motion
trajectory of the subject in a predefined
coordinate system — 202

reconstruct a final motion corrected MR image
from the measured k-space data and the
calculated motion trajectory in
the predefined coordinate system — 204

# Fig. 4

receive a training trial motion trajectory ⌐500

receive training trajectory data ⌐502

train the motion estimating neural network
using the training trial motion trajectory and
the training trajectory probability ⌐504

# Fig. 5

Fig. 6

EP 4 318 016 A1

$$\vec{k} = \begin{pmatrix} k_1 \\ \vdots \\ k_2 \end{pmatrix} \rightarrow \boxed{\phantom{x}} \rightarrow \cdots \rightarrow \boxed{\phantom{x}} \rightarrow p(\vec{k})$$

700

130

702

FC$_1$ 704 FC$_n$ 704

## Fig. 7

$$\vec{\beta} = \begin{pmatrix} \beta_1 \\ \vdots \\ \beta_n \end{pmatrix} \rightarrow \boxed{\phantom{x}} \rightarrow \cdots \rightarrow \boxed{\phantom{x}}$$

702

Conv$_1$ 802 Conv$_n$ 802

800

802 134

Conv $\rightarrow \theta(t)$

FC $\rightarrow p$ 130

704

## Fig. 8

900

802  802

$$\vec{\beta} = \begin{pmatrix} \beta_1 \\ \vdots \\ \beta_n \end{pmatrix}$$

Conv$_1$  ⋯  Conv$_n$  $\theta(t)$  134

702

FC  704

p  130

## Fig. 9

1000

802  802  134

$$\vec{\beta} = \begin{pmatrix} \beta_1 \\ \vdots \\ \beta_2 \end{pmatrix}$$

Conv$_1$  ⋯  Conv$_n$  $\theta(t)$

1002  802  1002  802  704

Pool$_1$  CONV$_1$  Pool$_2$  CONV$_2$  FC  p

702  130

## Fig. 10

**EP 4 318 016 A1**

<table>
<tr><td rowspan="2">Europäisches Patentamt<br>European Patent Office<br>Office européen des brevets</td><td rowspan="2" style="text-align:center">**EUROPEAN SEARCH REPORT**</td><td>**Application Number**</td></tr>
<tr><td>**EP 22 18 8017**</td></tr>
</table>

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | ALEXANDER LOKTYUSHIN ET AL: "Blind retrospective motion correction of MR images", MAGNETIC RESONANCE IN MEDICINE, vol. 70, no. 6, 11 February 2013 (2013-02-11), pages 1608-1618, XP055288829, US ISSN: 0740-3194, DOI: 10.1002/mrm.24615 * abstract * * figures 1, 6 * * section "METHODS" * ----- | 1-15 | INV. G01R33/565 G01R33/56 |
| A | WANG CHENGYAN ET AL: "Correction of out-of-FOV motion artifacts using convolutional neural network", MAGNETIC RESONANCE IMAGING, ELSEVIER SCIENCE, TARRYTOWN, NY, US, vol. 71, 25 May 2020 (2020-05-25), pages 93-102, XP086202735, ISSN: 0730-725X, DOI: 10.1016/J.MRI.2020.05.004 [retrieved on 2020-05-25] * abstract * * section "2. Theory" * * section "3. Methods" * ----- | 1-15 | |
| A | US 2022/189041 A1 (CAULEY STEPHEN [US] ET AL) 16 June 2022 (2022-06-16) * figures 1,2,3,6,7 * * paragraph [0014] – paragraph [0050] * ----- -/-- | 1-15 | TECHNICAL FIELDS SEARCHED (IPC) G01R G06N |

The present search report has been drawn up for all claims

| Place of search | Date of completion of the search | Examiner |
|---|---|---|
| Munich | 17 January 2023 | Durst, Markus |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**Application Number**

EP 22 18 8017

## EUROPEAN SEARCH REPORT

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | HAIKUN QI ET AL: "End-to-end deep learning nonrigid motion-corrected reconstruction for highly accelerated free-breathing coronary MRA", MAGNETIC RESONANCE IN MEDICINE, WILEY-LISS, US, vol. 86, no. 4, 6 June 2021 (2021-06-06), pages 1983-1996, XP071675394, ISSN: 0740-3194, DOI: 10.1002/MRM.28851 * section "2 ¦ METHODS" * | 1-15 | |
| A | LEE SEUL ET AL: "Deep Learning in MR Motion Correction: a Brief Review and a New Motion Simulation Tool (view2Dmotion)", INVESTIGATIVE MAGNETIC RESONANCE IMAGING, vol. 24, no. 4, 1 January 2020 (2020-01-01), page 196, XP093013416, ISSN: 2384-1095, DOI: 10.13104/imri.2020.24.4.196 Retrieved from the Internet: URL:https://pc.i-mri.org/pdf/10.13104/imri.2020.24.4.196> * the whole document * | 1-15 | TECHNICAL FIELDS SEARCHED (IPC) |

The present search report has been drawn up for all claims

| Place of search | Date of completion of the search | Examiner |
|---|---|---|
| Munich | 17 January 2023 | Durst, Markus |

EPO FORM 1503 03.82 (P04C01)

**ANNEX TO THE EUROPEAN SEARCH REPORT
ON EUROPEAN PATENT APPLICATION NO.**

EP 22 18 8017

This annex lists the patent family members relating to the patent documents cited in the above-mentioned European search report.
The members are as contained in the European Patent Office EDP file on
The European Patent Office is in no way liable for these particulars which are merely given for the purpose of information.

17-01-2023

| Patent document cited in search report | Publication date | Patent family member(s) | | Publication date |
|---|---|---|---|---|
| US 2022189041 A1 | 16-06-2022 | US | 2022189041 A1 | 16-06-2022 |
| | | WO | 2020198456 A1 | 01-10-2020 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **LUNDERVOLD.** An overview of deep learning in medical imaging focusing on MRI. *Z Med Phys,* 2019, vol. 29, 102-127